Europäisches Patentamt

European Patent Office

Office européen des brevets

(11) Publication number: **0 514 971 A1**

# EUROPEAN PATENT APPLICATION

(21) Application number: **92201344.6**

(22) Date of filing: **12.05.92**

(51) Int. Cl.5: **A61B 19/00, A61N 5/10**

(30) Priority: **22.05.91 GB 9111074**

(43) Date of publication of application:
**25.11.92 Bulletin 92/48**

(84) Designated Contracting States:
**DE FR GB**

(71) Applicant: **PHILIPS ELECTRONICS UK LIMITED**
**Philips House 1-19 Torrington Place**
**London WC1E 7HD(GB)**

(84) **GB**

(71) Applicant: **N.V. Philips' Gloeilampenfabrieken**

Groenewoudseweg 1
NL-5621 BA Eindhoven(NL)

(84) **DE FR**

(72) Inventor: **Driver, Brian Sydney**
**66 Langley Walk, Langley Green**
**Crawley, Sussex(GB)**

(74) Representative: **Clark, Jane Anne et al**
**PHILIPS ELECTRONICS Patents and Trade**
**Marks Department Philips House 1-19**
**Torrington Place**
**London WC1E 7HD(GB)**

(54) **Apparatus and a method for verifying a target position.**

(57) Apparatus (1) for verifying that a target position (10) within an area (11) of a patient (12) will be treated by a treatment beam (13) from a treatment machine (20), for example a radiotherapy machine. The coordinates of the target position (10) relative to a patient frame (14) secured to the treatment area (11) will have been previously determined by a diagnostic machine.

An irradiation beam detectable body (3) representing the target position (10) is supported by a moving arrangement (50) on a phantom frame (2) simulating the patient frame (14). The moving arrangement carries a position reference arrangement (60) for enabling the detectable body (3) to be moved to the determined coordinates relative to the phantom frame (2). The phantom frame (2) has a mounting arrangement (2a,2b) for mounting the phantom frame (2) to the treatment machine (20) precisely in place of the patient frame (14). The coordinates of the target position (10) can thus be verified using the actual machine (20) to be used for the subsequent treatment. This ensures greater accuracy and allows the target position (10) to be checked for different beam trajectories and patient orientations.

FIG.4

Rank Xerox (UK) Business Services

This invention relates to apparatus and a method for verifying that a target position within an area of a patient will be treated by a treatment beam from a treatment machine such as, for example, a radiotherapy machine.

Treatment by radiotherapy requires accurate alignment between the patient and the therapy machine to ensure that the target position is effectively irradiated to, for example, destroy cancerous cells with minimum damage to the surrounding healthy tissues. A recently developed technique known as stereotactic radiosurgery which effectively enables precise surgical operations to be carried out using a beam of radiation such as X-rays requires even greater precision, particularly when applied to the patient's head, because, in contrast to radiotherapy which may involve several sessions of treatment at given times over a period of weeks or months, radiosurgery is generally carried out in one session so that the effect of the treatment beam on healthy tissue surrounding the target position will be greater than in conventional radiotherapy.

In order to minimise the exposure to the treatment beam of healthy tissue surrounding the target position, various different systems exist for localising or identifying the target position in an unambigous manner and for correctly aligning the treatment beam with respect to the identified target position. The localising systems generally involve the use of some form of reference system attached to treatment area, for example the patient's skull. These may consist simply of a patient frame secured to the treatment area but may also include localising means in the form of radiation detectable markers or localising structures secured to the patient frame. The exact nature of the localising system will depend upon the diagnostic method, for example Computed Tomography, Magnetic Resonance Imaging, Angiography, used to detect the target position. Alignment may be carried out in a number of different ways, for example systems exist which use alignment lasers mounted to the treatment room and the treatment machine and some systems use X-ray alignment. By far the most accurate alignment system is the use of mechanical mountings on the patient frame and the treatment machine to correctly define the alignment of the target position and the treatment beam.

In order to provide further safeguards against unnecessary damage to healthy tissue surrounding the target volume, it is desirable to verify that the treatment beam will actually treat the target position after having completed the localisation and alignment procedures.

Where mechanical mountings on the patient frame and treatment machine are used to effect alignment of the treatment beam, a verification technique has been developed which is derived from the related field of neurosurgery. Such a verification system is used in the Philips SRS 200 Radiosurgery system and is described in the product data which accompanies that system. In outline, this verification system uses two separate devices. The first device, known as a phantom base, supports a frame simulating the patient frame and carries a movable pointer which is moved in three dimensions, using reference scales provided on the phantom base, to position the pointer at the coordinates of the target position (as determined from the preceding localising step) relative to the frame simulating the patient frame. The second device, known as a transfer means, is capable of being mounted on either the phantom base or to the treatment machine in place of the patient frame. The transfer means is first mounted to the frame of the phantom base and has its movable pointer adjusted to align with the pointer on the phantom base frame so that the tips of the pointers touch. The transfer means is then removed from the phantom base frame and its pointer replaced by a irradiation beam detectable sphere. The transfer means is then fitted to the treatment machine at precisely the location which would be occupied by the patient frame and the treatment beam is then used to determine the relative locations of the sphere and the field of the radiation beam to check whether or not the treatment beam is correctly aligned with the target position for various different orientations of the treatment beam. Although this system provides an independent check of the alignment of the treatment machine, the mounting of the transfer means on the phantom base, alignment of the pointers of the phantom base and transfer means and the alignment of the sphere and the pointer on the transfer means all provide areas of potential inaccuracy and increase the misalignment tolerances in the procedure.

It is an aim of the present invention to provide a more accurate method of verifying that a target position within an area of a patient will be treated by a treatment beam from a treatment machine such as a radiotherapy machine.

According to one aspect of the present invention, there is provided apparatus for verifying that a target position within an area of a patient will be treated by a treatment beam from a treatment machine, the coordinates of the target position relative to a patient frame secured to the treatment area having previously been determined, which apparatus comprises a phantom frame simulating the patient frame, an irradiation beam detectable body representing the target position, and moving means supporting the detectable body on the phantom frame and carrying position reference means for enabling the detectable body to be moved to the determined target position coordinates relative to the phantom frame, the phantom frame being provided with mounting means for mounting the phantom frame to the treatment machine precisely in the place of the patient frame for verifying that the treatment beam will treat the target position.

In a second aspect the present invention provides apparatus for directing a treatment beam at a target position within an area of a patient to be treated, the coordinates of the target position relative to a patient frame secured to the treatment area having previously been determined which apparatus comprises a treatment machine having a movable radiation source for providing the treatment beam, a support for supporting the patient to be treated and means for precisely locating the patient frame secured to the treatment area with respect to the radiation source, a phantom frame simulating the patient frame, an irradiation beam detectable body representing the target position and moving means supporting the detectable body on the phantom frame and carrying position reference means for enabling the detectable body to be moved to the determined target position coordinates relative to the phantom frame, the phantom frame being provided with mounting means for mounting the phantom frame to the locating means of the treatment machine precisely in the place of the patient frame to enable a user to use the treatment machine verify that the treatment beam will treat the target position.

The present invention also provides a method for verifying that a target position within an area of a patient will be treated by a treatment beam from a treatment machine, the coordinates of the target position relative to a patient frame secured to the treatment area having previously been determined, which method comprises positioning an irradiation detectable body supported by moving means on a phantom frame simulating the patient frame at the determined coordinates relative to the phantom frame using position reference means carried by the moving means and mounting the phantom frame to the treatment machine precisely in the place of the patient frame and then detecting the position of the detectable body by irradiating the phantom frame using the treatment machine.

Apparatus and a method in accordance with the invention thus allow the irradiation detectable body to be set precisely at the coordinates of the target position relative to the phantom frame directly using the moving means and position reference means carried by the moving means and allow the phantom frame simulating the patient frame to be fixed to the treatment machine in precisely the same location as the location in which the patient frame will be fixed during treatment, whilst avoiding the inaccuracies which may be introduced into the procedure when using the known phantom base and transfer means. The coordinates of the target position as represented by the detectable body can thus be verified using the treatment machine itself. This not only ensures greater accuracy but also allows the verification of the target position to be checked for different trajectories of the treatment beam. This is particularly important because during radiotherapy or radiosurgery it is generally desirable to irradiate the target position from a number of different positions and angles to ensure maximum exposure of the target position compared with minimum exposure of the surrounding healthy tissue through which the treatment beam has to pass.

The treatment machine may be provided with means for providing an image of the detectable body relative to the treatment beam to provide a film record for the verification. The means for providing the image may be movable with the radiation source to provide images over the full range of movement of the radiation source.

The locating means of the treatment machine may comprise a fixedly located support column positioned adjacent the patient support, a mounting member providing mounting points to which a patient frame is to be mounted and adjustment means connecting the support column to the mounting member to enable a patient frame to be moved to precisely locate the target position. The adjustment means enables accurate alignment of the phantom and patient frames with respect to the radiation source. Alternatively, the locating means may be provided in the patient support although this would generally be a less accurate arrangement.

The phantom frame may have further mounting means for receiving a localising means previously secured to the patient frame to enable the relative position of the localising means and the detectable body to be checked. This enables the alignment of the localising means with respect to the detectable body to be verified using the diagnostic machine previously used to locate the target position which should further increase the accuracy of the target position determination.

The moving means may comprise a first member mounted to the phantom frame so as to be movable in a first direction relative to the phantom frame, a second member mounted to the first member so as to be movable in a different second direction relative to the first member and a third member mounted to the second member so as to be movable in a third direction different from the first and second directions, the third member carrying the radiation detectable body. The position reference means may comprise Vernier-type scales for indicating the respective locations of the first, second and third members.

In a preferred example, the moving means may comprise a first carriage movable in a first direction along a first track, second and third carriages fixed to respective opposite ends of the first track, each of the second and third carriages being movable in a second direction perpendicular to the first direction along a respective one of two parallel second tracks and a support member carrying the detectable body, the support member being mounted to the first carriage so as to be movable relative to the first carriage in a third direction perpendicular to the first and second directions. The carriages may comprise micrometres forming the position reference means for indicating the respective locations of the carriages on their respective tracks and the location of the support member relative to the first carriage. This relatively simple arrangement allows accurate movement of the irradiation detectable body in three dimensions. Each of the carriages and the support member may be movable by means of a respective motorised drive means.

Generally, the detectable body will be an X-ray opaque sphere. The use of a sphere has the advantage that it represents the same outline whatever the relative orientation of the sphere and the treatment beam and facilitates an accurate determine of the alignment with the treatment beam especially where the treatment beam is of circular cross-section.

Embodiments of the invention will now be described, by way of example, with reference to the accompanying drawings, in which

Figure 1 is a side view of a radiotherapy machine with which apparatus in accordance with the invention is to be used;

Figure 2 is a plan view of apparatus in accordance with the invention for verifying a target position within an area of a patient to be treated by a treatment beam;

Figure 3 is a sectional view, taken along the line III-III in Figure 2 of the apparatus shown in Figure 2;

Figure 4 is a side view similar to Figure 1 but with a phantom frame of the apparatus shown in Figure 2 attached to the radiotherapy machine in place of a patient frame;

Figure 5 is a view taken along the line V-V in Figure 4 of a collimator head of the radiotherapy machine and the detectable body for illustrating the manner in which the target position can be verified using the apparatus shown in Figures 2 and 3;

Figure 6 shows a typical angiographic localising means mounted to the apparatus shown in Figures 2 and 3; and

Figure 7 shows a typical CT (Computerised Tomography) localising means mounted to the apparatus shown in Figures 2 and 3.

It should of course be understood that the Figures are very diagrammatic and are not drawn to scale.

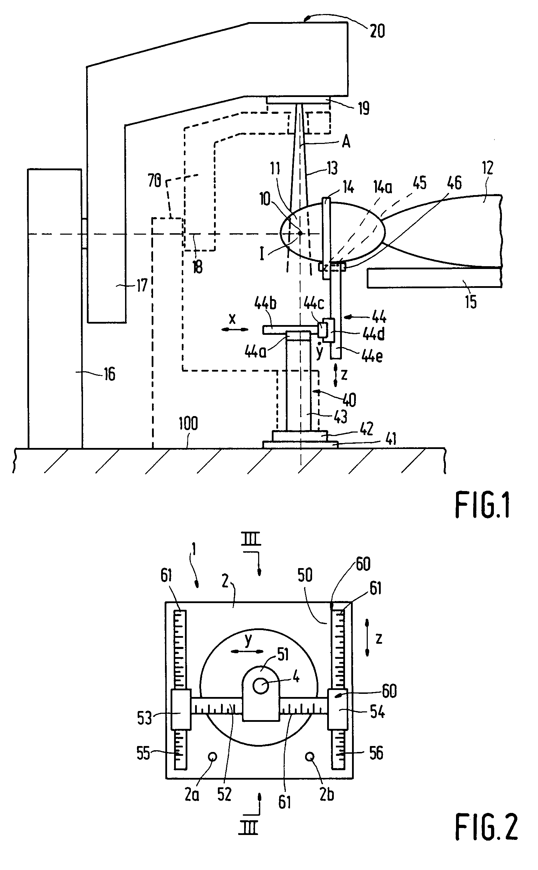

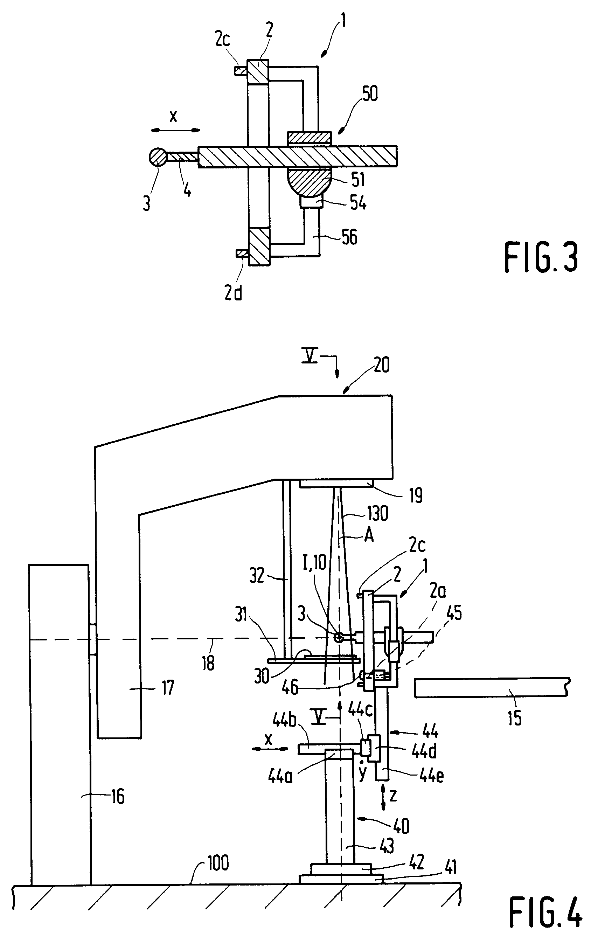

Referring now to the drawings, in particular Figures 2 to 4, there is illustrated apparatus 1 for verifying that a target position 10 within an area 11 of a patient 12 will be treated by a treatment beam 13 from a treatment machine 20, in the example shown a radiotherapy machine. The coordinates of the target position 10 relative to a patient frame 14 secured to the treatment area 11 will have been previously identified or determined. As shown most clearly in Figures 2 and 3, the apparatus 1 comprises a phantom frame 2 simulating the patient frame 14, an irradiation beam detectable body 3 representing the target position 10 and moving means 50 supporting the detectable body 3 on the phantom frame 2 and carrying position reference means 60 for enabling the detectable body 3 to be moved to the determined target position coordinates relative to the phantom frame 2, the phantom frame 2 being provided with mounting means 2a,2b for mounting the phantom frame 2 to the treatment machine 20 precisely in place of the patient frame 14 for verifying that the treatment beam 13 will treat the target position 10.

The apparatus shown in Figures 2 and 3 allows the irradiation detectable body 3 representing the target position 10 to be set precisely at the coordinates of the target position 10 relative to the phantom frame 2 directly using the moving means 50 and position reference means 60 carried by the moving means 50 and allows the phantom frame 2 simulating the patient frame 14 to be fixed to the treatment machine 20 in precisely the same location as the location in which the patient frame 14 will be fixed during treatment whilst avoiding the inaccuracies inherent in the use of the known phantom base and transfer means. The coordinates of the target position 10, as represented by the detectable body 3, can thus be verified using the actual machine to be used for the subsequent treatment. This ensures greater accuracy and allows the verification of the target position 10 to be checked for different trajectories of the treatment beam 13 and for different orientations of the patient 12. Generally in radiotherapy or radiosurgery it is desirable to irradiate the target position from a number of different positions and angles to ensure maximum exposure of the target position whilst keeping the exposure to the treatment beam of the surrounding healthy tissue as low as possible. Because apparatus in accordance with the invention is mounted to the actual treatment machine to be used for the subsequent treatment, the determined location of the target position 10 can be checked throughout the range of trajectories proposed by the conventional treatment planning for the treatment beam. This enables the determined target coordinates to be verified for all stages of the subsequent treatment.

Referring now specifically to the drawings, Figure 1 illustrates very schematically a radiotherapy machine 20 with which apparatus 1 in accordance with the invention may be used to verify a determined target position 10 within a treatment area 11. The radiotherapy machine 20 is designed to irradiate the target position 10 within the treatment area 11 of a patient 12 with a collimated beam of X-rays or possibly electrons. It should of course be understood that the treatment beam may comprise any suitable radiation and that moreover the treatment beam may be formed by several subsidiary beams focussed onto the the desired target positin as in the so-called Gamma knife.

The radiotherapy machine 20 comprises a support 16 carrying a gantry 17 which is rotatable through substantially 360° with respect to the support 16 about a generally horizontal axis 18. Although not shown in Figure 1, the gantry contains, in series, an electron source, a linear accelerator (linac) for accelerating electrons from the source to an energy typically in the range of from 4 to 25 MeV (mega-electron volts) and a beam deflection system which gives the electron beam a net deflection of more than 90° (degrees) so that the beam is directed normally of and towards the axis 18. The gantry 17 carries a collimator head 19 for collimating the deflected beam of electrons to provide the treatment beam. A suitable X-ray target may be provided after the deflection system so as to enable the radiotherapy machine 20 to produce a collimated beam of X-rays rather than electrons. The radiotherapy machine 20 itself is of generally conventional design and may be, for example, an SL25 manufactured by Philips Medical Systems at Crawley, UK.

Figure 1 also shows part of a patient support in the form of an adjustable table 15 upon which the patient 12 is placed. A patient frame 14 is fixed to the treatment area 11 of the patient 12. In this example, the patient frame 14 is in the form of a head ring surrounding and fixedly secured by conventional means, for example by screws inserted into the skull. The patient frame 14 is used to enable a precise and consistent relationship to the target position 10 to be achieved.

The target position 10 is determined by providing localising means on the patient frame 14 and by scanning the treatment area 11 using, for example, conventional angiographic, MR (Magnetic Resonance) or CT (Computerised Tomography) diagnostic scanning techniques to determine the coordinates of the target position 10 with respect to the patient frame 14. The localising means are, or have portions which are, opaque to the scanning beam and so provide reference markers on the scan image to enable a precise identification of the target position 10 from which information an effective treatment plan giving the trajectories, doses etc. of the treatment beams is required to treat the target position 10 (which may be, for example a tumour) can be determined using known computer treatment planning techniques.

In order to ensure that the target position 10 receives maximum exposure to the treatment beam(s) 13 whilst minimising the exposure and thus damage to the normally healthy tissue, the target position 10 should be precisely positioned at the isocentre I of the radiotherapy machine 20, that is the point at which the axis of the treatment beam 13 intersects the axis 18 of rotation of the gantry 17. For this purpose, an adjustable stand 40 is provided. The stand 40 is mounted on the same reference surface or floor 100 as the support 16 and is arranged at a precise location with respect to the support 16.

In order to improve even further the accuracy of alignment of the system, the stand 40 may be directly coupled to the collimator head 19 via a gimbal mounting arrangement indicated schematically by the phantom-line block referenced 70 in Figure 1. This gimbal mounting arrangement 70 may be similar to that described in International Patent Application Publication No. WO89/05171 or that used in the Philips SRS 200 Radiosurgery system.

The stand 40 comprises a base 41 providing a bearing 42 for a rotatable column 43 so that the column 43 is rotatable about its own generally vertical axis 43a which is perpendicular to the axis 18 and is aligned with the axis A of the treatment beam 13. The column 43 carries adjustment means 44 which allow movement in each of three perpendicular directions.

In the example shown the adjustment means 44 is in the form of an XYZ carriage arrangement. The adjustment means shown in Figure 1 comprises a first carriage 44a provided on the column 43 and in which a first support member 44b is movable in a first direction X, a second support member 44c fixed at one end of the first support member 44b so as to extend in a second direction Y perpendicularly of the first direction X, a second carriage 44d movable along the second support member 44c in the second direction Y and a third support member 44e mounted to the second carriage 44d so as to be movable in a third direction Z, perpendicular to the first and second directions X and Y, relative to the second carriage 44d. The third support member 44e is provided with two precisely positioned mounting points, in the example shown apertures 45, to which the patient frame 14 is attached by means of screws or bolts 46 passing through apertures 14a in the patient frame 14 aligned with the apertures 45. The XYZ carriage arrangement 44 allows the position of the patient frame 14, and thus the target position 10, to be adjusted to within 0.1mm (millimetres) to enable the target position 10 to be located precisely at the isocentre I before treatment by irradiation with the treatment beam 13 commences.

During treatment, the gantry 17 is rotated so as to alter the location and/or angle of the treatment beam 13 so, as mentioned above, to minimise the irradiation of normally healthy tissue.

Before treatment commences it is necessary to verify that the determined target position coordinates obtained using one of the above described diagnostic scanning techniques are correct. Figures 2 and 3 illustrate schematically one example of apparatus in accordance with the invention for effecting such verification.

As shown in Figure 2, the apparatus 1 comprises a phantom frame 2 which simulates the patient frame 14. Thus, the phantom frame 2 is formed to have precisely the same critical dimensions as the patient frame 14. Thus, the phantom frame 2 has two mounting points 2a,2b which are arranged to correspond precisely to the positions of the mounting points 14a of the patient frame 14. In this example, the mounting points 2a,2b are precision machined apertures which align precisely with the apertures 45 of the support member 44e and allow the phantom frame 2 to be secured by the fixing screws 46 to the support member 44e so that the phantom frame 2 ocupies exactly the same position as the patient frame 14 normally would. Figure 4 illustrates the phantom frame 2 mounted to the support member 44e.

The phantom frame 2 carries an irradiation beam detectable body 3 in the form of an X-ray opaque, in this example, sphere on a support rod 4 which is connected to the phantom frame 2 via moving means 50 which allows the sphere 3 to be moved in three dimensions relative to the phantom frame 2 to allow the sphere 3 to be positioned relative to the mounting points 2a, 2b of the phantom frame 2 so that the relative positions of the sphere 3 and mounting points 2a, 2b are identical to those of the target position 10 and the mounting points 14a of the patient frame 14.

The moving means 50 comprises in this example an XYZ carriage arrangement.

As shown in Figure 2, the moving means 50 comprises a first carriage 51 movable in a first direction Y along a first track 52. Second and third carriages 53 and 54 are fixed to opposite ends of the track 52. Each of the second and third carriages 53 and 54 is movable in a second direction Z perpendicular to the first direction Y along a respective one of two parallel tracks 55 and 56 mounted to the phantom frame 2. The support rod 4 is slidably received within a bearing provided in the first carriage 51 so as to enable the support rod 4 to move in a third direction X perpendicular to the first and second directions X and Z. The support rod 4 may be mounted to the first carriage 51 by any suitable means for example by means of a slide mounted to the first carriage 51.

Any suitable means for moving the carriages 51,53 and 54 relative to the tracks 52,55 and 56 and for moving the support rod 4 relative to the first carriage 51 may be provided. Indeed the carriages 51,53 and 54 may be manually movable. In this example, the movement may be motor driven and the carriages 51,53,54 and tracks 52,55,56 and support rod 4 may have a worm drive or rack and pinion arrangement. It is desired that the detectable body 3 be movable within a range +100mm to -60mm about the isocentre I along the beam axis (that is the Z direction) and within a range of from +100mm in the lateral (that is the Y) and the AP (Anterior-Posterior) (that is the X) directions to an accuracy of 0.1mm. The moving means 50 carries position reference means 60 for precisely identifying the location of the carriages 51,53 and 54. The position reference means may be provided by any scale, for example a Vernier scale, capable of providing the required accuracy. In this example each of the carriages 51,3 and 54 comprises a digital display micrometer, for example of the type manufactured by Mitutoyo of Japan, and each track 52,55 and 56 and the support and 4 carries a scale 61 divided into at least 0.1mm divisions for enabling calibration and zeroing of the micrometers. In addition, although not shown, locating means should be provided to fix the position of the carriages 51,53,54 with respect to the tracks 52,55,56 and support rod 4, as appropriate, when the detectable body or sphere 3 has been positioned at the determined target position 10.

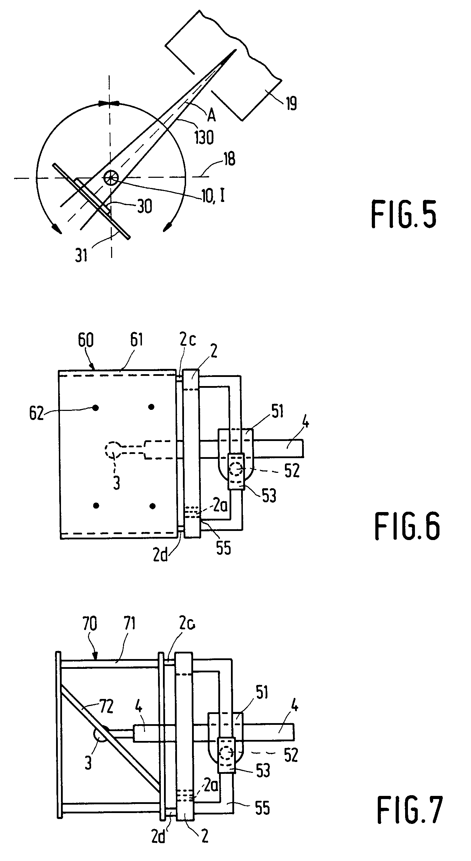

In use of the apparatus 1 shown in Figures 2 and 3 the phantom frame 2 is first mounted to the moving means 44 of the stand 40 by aligning the apertures 2a,2b with the apertures 45a and inserting the locking screws 46. As the phantom frame 2 simulates the patient frame 14 precisely and the coordinates of the target position 10 have previously been determined with respect to the patient frame 14 using one of the diagnostic techniques mentioned above, the determined coordinates of the target position 10 can then be used to set, using the moving means 50, the sphere 3 at the determined location of the target position 10 relative to the phantom frame 2. Alternatively, the sphere 3 may be set at the target position 10 before the phantom frame 2 is mounted to the moving means 44 of the stand 40. Once this has been done and the sphere 3 has been locked in place at the determined location of the target position 10, the adjustment means 44 is used to position the apparatus 1, and thus the sphere 3, at the required location with respect to the treatment beam 13 as determined during the alignment procedure that is so that the sphere 3 is positioned at the isocentre I. The radiotherapy machine is then adjusted to provide an X-ray radiation beam 130 which is directed at the sphere 3 in exactly the same manner as the treatment beam 13 is to be directed at the target position 10. Indeed the irradiation beam 130 may be identical in all respects to the treatment beam 13. The irradiation beam should in any case have an axis which is precisely coincident with the axis A of the treatment beam. The gantry 17 can then be rotated to move the irradiation beam 130 to each of the settings for the treatment beam previously determined by conventional treatment planning. The patient support 15 may also be moved to simulate the desired treatment settings. Figure 5 illustrates schematically an example of such setting.

As shown in Figure 4, a film 30 may be supported on a mount 31 secured to the gantry 17 by a support member 32 so that the film 30 is positioned directly behind the sphere 3. Thus, images of the sphere irradiated by the irradiation beam 130 at the various settings for the treatment beam 13 can be taken and used to check that the sphere 3 is at the centre of the, generally, circular field of the radiation beam 130 so ensuring that the target position 10 will similarly be at the centre, i.e. on the axis A, of the treatment beam 13.

If the above-described verification procedure is satisfactory, then the treatment of the patient 12 may commence. Accordingly, with the patient 12 supported on the adjustable table 15 at the appropriate height and with the patient frame 14 mounted to the mounting points 45 by, in this example, the screws or bolts 46 passing through the aligned apertures 14a and 45, the setting of the XYZ carriage arrangement 44 which was fixed prior to the verification procedure should result in the target position 10 being located at the isocentre I of the radiotherapy machine 20. The treatment of the patient can then begin in accordance with the calculated treatment plan. If, however, the verification procedure does not produce satisfactory results it will be necessary to identify the error in the set up, for example, by confirming that the adjustment means 44 and the moving means 50 have both been correctly set.

In order to improve even further the accuracy of the target position 10 location determination and/or to assist in assessing where the problems may lie if the verification is not satisfactory, the phantom frame 2 is preferably provided with further mounting means 2c,2d which enable the localising means used during the diagnostic scan to be mounted to the phantom frame 2 to allow the alignment between the localising means and the sphere 3 (and thus the target position 10) to be verified. Preferably, this verification is carried out using the diagnostic machine originally used during the localisation of the target position so to eanble a more accurate detection of the fiducial markers provided by the localising means. The further mounting means 2c,2d simulate the localising means mounting points 14b of the patient frame 14 and are precisely positioned so that the localising means is in precisely the same relationship to the phantom frame 2 as it was to the patient frame 14. Figure 6 illustrates schematically a typical angiographic localising means 60 consisting of, for example, a polymethyl methacrylate (PMMA) such as PERSPEX (Registered Trade Mark) surface or surfaces 61 provided with radioopaque markers 62 mounted to the further mounting means 2c,2d of the phantom frame 2 whilst Figure 7 illustrates schematically a typical CT localising means 70 consisting of a parallelopipedal arrangement of X-ray opaque rods 71 having diagonal X-ray opaque rods 72 which enable relative positions to be determined mounted to the phantom frame 2.

Apparatus in accordance with the invention thus enables a radiation source of a treatment machine such as a radiotherapy machine to be positioned precisely relative to a target position to enable a treatment beam subsequently to be directed precisely at the target position to, for example, expose a tumour at the target position to irradiation which will destroy or at least partially destroy the tumour whilst minimising the exposure of the healthy tissue surrounding the tumour.

Although in the example described above, the moving means 50 forms a rectilinear coordinate system, other coordinate systems may be used for the moving means. Thus, for example a polar coordinate system could be used for which the moving means has a first circularly annular member mounted in an appropriate bearing to the phantom frame so as to be movable in a first arcuate direction and a second circularly arcuate member mounted to the first member in a suitable bearing so as to be movable relative to the first member in a second arcuate direction. The support rod 4 may then be mounted to the second member so as to be movable relative to the second member in a radial direction with respect to the second member. Such an arrangement may be more compact that the moving means 50 described above and in addition the phantom frame could then be reduced to a support bar carrying the mounting parts 2a and 2b (and 2c and 2d if present) and the first member. The use of a polar coordinate system could be of advantage in reducing the risk of the introduction of systematic errors because the alignment coordinates for the treatment machine will generally be rectilinear coordinates and a systematic error in the reading of the treatment machine rectilinear coordinates could not be easily repeated when setting the polar coordinates on the moving means.

From reading the present disclosure, other modifications and variations will be apparent to persons skilled in the art. Such modifications and variations may involve other features which are already known in the radiotherapy art and which may be used instead of or in addition to features already described herein. Although claims have been formulated in this application to particular combinations of features, it should be understood that the scope of the disclosure of the present application also includes any novel feature or any novel combination of features disclosed herein either explicitly or implicitly, whether or not it relates to the same invention as presently claimed in any claim and whether or not it mitigates any or all of the same technical problems as does the present invention. The applicants hereby give notice that new claims may be formulated to such features and/or combinations of such features during the prosecution of the present application or of any further application derived therefrom.

## Claims

1. Apparatus for verifying that a target position within an area of a patient will be treated by a treatment beam from a treatment machine, the coordinates of the target position relative to a patient frame secured to the treatment area having previously been determined, which apparatus comprises a phantom frame simulating the patient frame, an irradiation beam detectable body representing the target position, and moving means supporting the detectable body on the phantom frame and carrying position reference means for enabling the detectable body to be moved to the determined target position coordinates relative to the phantom frame, the phantom frame being provided with mounting means for mounting the phantom frame to the treatment machine precisely in the place of the patient frame for verifying that the treatment beam will treat the target position.

2. Apparatus for directing a treatment beam at a target position within an area of a patient to be treated, the coordinates of the target position relative to a patient frame secured to the treatment area having previously been determined which apparatus comprises a treatment machine having a movable radiation source for providing the treatment beam, a support for supporting the patient to be treated and means for precisely locating the patient frame secured to the treatment area with respect to the radiation source, a phantom frame simulating the patient frame, an irradiation beam detectable body representing the target position and moving means supporting the detectable body on the phantom frame and carrying position reference means for enabling the detectable body to be moved to the determined target position coordinates relative to the phantom frame, the phantom frame being provided with mounting means for mounting the phantom frame to the locating means of the treatment machine precisely in the place of the patient frame to enable a user to use the treatment machine verify that the treatment beam will treat the target position.

3. Apparatus according to Claim 2, wherein the treatment machine is provided with means for forming an image of the detectable body relative to the treatment beam.

4. Apparatus according to Claim 2 or 3, wherein the locating means of the treatment machine comprises a fixedly located support column positioned adjacent the patient support, a mounting member providing mounting points to which a patient frame is to be mounted and adjustment means connecting the support column to the mounting member to enable a patient frame to be moved relative to the support column.

5. Apparatus according to Claim 1,2,3 or 4, wherein the phantom frame has further mounting means for receiving a localising means previously secured to the patient frame to enable the relative positions of the localising means and the detectable body to be verified.

6. Apparatus according to any one of the preceding Claims, wherein the moving means comprises a first member mounted to the phantom frame so as to be movable in a first direction relative to the phantom frame, a second member mounted to the first member so as to be movable in a different second direction relative to the first member and a third member mounted to the second member so as to be movable in a third direction different from the first and second directions, the third member carrying the radiation detectable body.

7. Apparatus according to Claim 6, wherein the position reference means comprise Vernier-type scales for indicating the respective locations of the first, second and third members.

8. Apparatus according to any one of Claims 1 to 5, wherein in that the moving means comprises a first carriage movable in a first direction along a first track, second and third carriages fixed to respective opposite ends of the first track, each of the second and third carriages being movable in a second direction perpendicular to the first direction along a respective one of two parallel second tracks and a support member carrying the detectable body, the support member being mounted to the first carriage so as to be movable relative to the first carriage in a third direction perpendicular to the first and second directions.

9. Apparatus according to Claim 8, wherein the carriages comprise micrometres forming the position reference means for indicating the respective locations of the carriages on their respective tracks and the location of the support member relative to the first carriage.

10. Apparatus according to Claim 8 or 9, wherein each of the carriages and the support member is movable by means of a respective motorised drive means.

11. Apparatus according to any one of the preceding claims further characterised in that the detectable body comprises an X-ray opaque sphere.

12. A method for verifying that a target position within an area of a patient will be treated by a treatment beam from a treatment machine, the coordinates of the target position relative to a patient frame secured to the treatment area having previously been determined, which method comprises positioning an irradiation detectable body supported by moving means on a phantom frame simulating the patient frame at the determined coordinates relative to the phantom frame using position reference means carried by the moving means and mounting the phantom frame to the treatment machine precisely in the place of the patient frame and then detecting the position of the detectable body by irradiating the phantom frame using the treatment machine.

13. A method according to Claim 12, further comprising mounting a localising means previously secured to the patient frame to further mounting means provided on the phantom frame to verify the relative positions of the localising means and the detectable body.

FIG.1

FIG.2

FIG.3

FIG.4

FIG.5

FIG.6

FIG.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-4 706 665 (K.I.GOUDA) | 1,2 | A61B19/00 |
| A | * column 4, line 4 - column 9, line 2; figures 1,2 * | 5-9,12 | A61N5/10 |
| | --- | | |
| Y | MEDICAL PHYSICS<br>vol. 12, no. 4, July 1985, NEW YORK, US<br>pages 469 - 472;<br>P.V.HOUDEK ET AL: 'Stereotaxic radiotherapy technique for small intracranial lesions'<br>* page 469, left column, line 24 - page 471, right column, line 8; figures 1-4 * | 1,2 | |
| | --- | | |
| D,A | WO-A-8 905 171 (UNIVERSITY OF FLORIDA)<br>* page 14, line 30 - page 17, line 14; figures 1-4 * | 1-4 | |
| | ----- | | |
| | | | TECHNICAL FIELDS SEARCHED (Int. Cl.5 ) |
| | | | A61B<br>A61N |

The present search report has been drawn up for all claims

| Place of search | Date of completion of the search | Examiner |
|---|---|---|
| BERLIN | 19 AUGUST 1992 | WEIHS J. |

EPO FORM 1503 03.82 (P0401)